(19) Europäisches Patentamt
European Patent Office
Office européen des brevets

(11) Veröffentlichungsnummer: **0 287 773**
**A1**

# (12) EUROPÄISCHE PATENTANMELDUNG

(21) Anmeldenummer: **88102552.2**

(51) Int. Cl.⁴: **A61K 7/09 , A61K 7/155**

(22) Anmeldetag: **22.02.88**

(30) Priorität: **23.04.87 DE 3713559**

(43) Veröffentlichungstag der Anmeldung:
**26.10.88 Patentblatt 88/43**

(84) Benannte Vertragsstaaten:
**AT BE CH DE ES FR GB GR IT LI NL SE**

(71) Anmelder: **Wella Aktiengesellschaft**
**Berliner Allee 65**
**D-6100 Darmstadt(DE)**

(72) Erfinder: **Götz, Harry**
**Waldstrasse 21**
**D-6146 Alsbach-Hähnlein 2(DE)**
Erfinder: **Hartmann, Peter**
**Hoffmannstrasse 6**
**D-6100 Darmstadt(DE)**
Erfinder: **Köhler, Joachim, Dr.**
**Waldstrasse 51**
**D-6101 Brensbach/Odenw.(DE)**

## (54) Mittel zur dauerhaften Verformung oder zur Entfernung von Haaren.

(57) Die Erfindung betrifft ein kosmetisches Mittel zur dauerhaften Verformung oder zur Entfernung von Haaren auf der Basis eines keratinerweichenden oder oxidierenden Wirkstoffes, welches dadurch gekennzeichent ist, daß es 0,01 bis 1,2 Gewichtsprozent einer Siliciumdioxid/Dimethylpolysiloxan-Mischung enthält.

Das erfindungsgemäße Mittel besitzt eine gegenüber üblichen derartigen Mitteln deutlich gesteigerte Wirksamkeit bei gleichguter physiologischer Verträglichkeit.

EP 0 287 773 A1

Xerox Copy Centre

## Mittel zur dauerhaften Verformung oder zur Entfernung von Haaren

Die vorliegende Erfindung betrifft ein eine Siliciumdioxid/ Dimethylpolysiloxan-Mischung enthaltendes Mittel zur dauerhaften Verformung oder zur Entfernung von Haaren.

Für die dauerhafte Verformung von menschlichen Haaren werden die Haare zunächst mit einem das Haarkeratin erweichenden Haarverformungsmittel behandelt. Man verwendet hierzu im allgemeinen Alkalihydroxide oder reduzierend wirkende Schwefelverbindungen, die entweder zur Gruppe der Sulfide, Sulfite beziehungsweise Hydrogensulfite oder zur Gruppe der Mercaptocarbonsäuren gehören. Diese Substanzen sind imstande, das Keratin des menschlichen Haares in seiner Struktur zu erweichen und eine Verformung der Haare zu ermöglichen.

Im Anschluß an diese Erweichung des Haarkeratins wird das Haar mit Wasser gespült und im Falle einer Verformungsbehandlung mit reduzierend wirkenden Verformungsmitteln durch Behandlung mit einem Verformungsmittel auf der Basis eines oxidierenden Wirkstoffes, vorzugsweise Bromat oder Wasserstoffperoxid, fixiert. Hierbei werden die zuvor gespaltenen Disulfidbrückenbindungen des Haarkeratins in der dem Haar verliehenen neuen Form wiederverknüpft. Anschließend wird das Haar mit Wasser gespült.

Die chemische Entfernung von Haaren (Depilation) erfolgt in ähnlicher Weise wie die dauerhafte Haarverformung durch Behandlung der Haare mit einem keratinerweichenden Wirkstoff. Infolge eines stärker alkalischen pH-Wertes (pH = 11 bis 13) verläuft bei der Depilation der Prozeß der Quellung des Haares und die Spaltung der Disulfidbrückenbindungen des Haarkeratins bis zur völligen Auflösung der Haare.

Die vorstehend beschriebenen Verfahren zur dauerhaften Verformung oder zur Entfernung von Haaren weisen jedoch erhebliche Nachteile auf. So ist es zur Erzielung einer ausreichenden Wirkung oft erforderlich, die genannten keratinerweichenden Wirkstoffe in einer relativ hohen Konzentration einzusetzen oder die Anwendungsdauer zu verlängern. Ferner sind bestimmte keratinerweichende Wirkstoffe, wie zum Beispiel Sulfite und Monothioglykolsäureester, in Haarverformungsmitteln, welche auf einen pH-Wert von etwa 6 eingestellt sind, erst bei gleichzeitiger Anwendung von Temperaturen oberhalb von 60 Grad Celsius in ausreichendem Maße wirksam. Alkalithyoglykolate entfalten in Enthaarungsmitteln erst oberhalb einer Thioglykolsäurekonzentration von 3 Gewichtsprozent eine ausreichende Wirksamkeit, wobei diese Wirksamkeit durch Erhöhung der Thioglykolsäurekonzentration (auf bis zu 10 Gewichtsprozent) nur sehr geringfügig gesteigert werden kann.

Durch die Erhöhung der Temperatur oder der Wirkstoffkonzentration sowie durch eine Verlängerung der Einwirkungszeiten wird die physiologische Verträglichkeit der verwendeten kosmetischen Mittel erheblich beeinträchtigt. Die Folgen können Hautirritationen, Brennerscheinungen und in seltenen Fällen sogar Verätzungen der Haut oder Kopfhaut sein.

Man hat daher stets nach Möglichkeiten gesucht, bei derartigen kosmetischen Mitteln durch eine entsprechende Zusammensetzung und Anwendungsweise eine mög lichst starke Wirksamkeit bei gleichzeitig guter physiologischer Verträglichkeit zu erreichen. Was die physiologische Verträglichkeit betrifft, so werden bezüglich der Zusammensetzung der Präparate geringe Wirkstoffkonzentrationen und die Einstellung auf haar-beziehungsweise hautverträgliche pH-Werte angestrebt. Gleichzeitig ist man hinsichtlich ihrer Anwendung um kürzere Einwirkungszeiten und das Arbeiten bei Raumtemperatur bemüht.

Damit man mit solchen Präparaten dennoch eine starke Wirksamkeit bei gleichzeitiger guter physiologischer Verträglichkeit erzielen kann, werden diesen oft Stoffe zugesetzt, die das Eindringen der Wirkstoffe in das Skleroprotein des Haares begünstigen und die Wirksamkeit der Präparate, in denen sie enthalten sind, erhöhen.

Beispielsweise läßt sich bei Verwendung alkalischer Präparate durch Zusatz dieser Verbindungen eine gute Haarverformung erzielen, auch wenn das Verformungsmittel nur eine vergleichsweise geringe Konzentration an keratinerweichendem Wirkstoff enthält. Weiterhin ist es durch den Zusatz dieser Verbindung zu Enthaarungsmitteln möglich, die für eine Haarentfernung erforderlichen Einwirkungszeiten zu verkürzen.

Die in kosmetischen Mitteln zur dauerhaften Verformung oder zur Entfernung von Haaren bisher verwendeten wirkungsverstärkenden Stoffe weisen jedoch verschiedene Nachteile auf, so daß die erzielten Ergebnisse nicht immer zufriedenstellend sind. So sind diese Stoffe entweder in Wasser schwer löslich, wie beispielsweise Melamin, oder aber physiologisch unverträglich, was zum Beispiel für Formamid zutrifft. Andere derartige Stoffe, wie beispielsweise Alkali-und Ammoniumthiocyanate, werden durch eine nachfolgende Oxidationsbehandlung unter Bildung unerwünschter Nebenprodukte zersetzt oder sie sind relativ leicht verseifbar, wie dies zum Beispiel bei Harnstoff der Fall ist. Alkohole, wie beispielsweise Isopropanol, setzen die Hautverträglichkeit des Mittels herab.

Verwendet man zum Beispiel Harnstoff in einem sauren Haarverformungsmittel (pH = 6,0) auf

Sulfitbasis, so steigt der pH-Wert des Mittels während der Lagerung durch Verseifung des Harnstoffes unter Bildung von Ammoniumcarbonat allmählich auf einen pH-Wert von 7 oder darüber an, so daß die Verformungswirksamkeit des Mittels verlorengeht. Setzt man den Harnstoff in einem alkalischen Enthaarungsmittel vom pH 12 auf der Basis einer Mercaptoverbindung ein, so erfolgt ebenfalls allmählich eine Verseifung des Harnstoffes unter Bildung von Ammoniumcarbonat. Hierbei wird jedoch sekundär die Kohlensäure durch das Alkali des Mittels gebunden und somit infolge einer Senkung des pH-Wertes die Wirksamkeit des Enthaarungsmittels verringert.

Es wurde bereits versucht, die vorerwähnten Nachteile durch den Einsatz von neuen, wirkungssteigernden Stoffen, wie zum Beispiel Imidazolidin-2-on, zu beheben. In diesem Zusammenhang wird Bezug genommen auf die eigene deutsche Patentschrift 26 14 723. Aufgrund neuerer Untersuchungen sind jedoch Bedenken gegen die physiologische Verträglichkeit des Imidazolidin-2-ons erhoben worden.

Ein weiteres Problem des vorstehend beschriebenen Verfahrens zur dauerhaften Haarverformung ist ein schlechter Halt der Frisur infolge einer ungenügenden oxidativen Fixierung.

Alle Versuche, eine schnelle und zuverlässige Fixierung bei gleichzeitig niedriger Konzentration an oxidierendem Wirkstoff zu erzielen, konnten bisher nicht befriedigen.

Es bestand daher die Aufgabe, die Wirkung der vorstehend genannten kosmetischen Mittel zur Verformung oder zur Entfernung von Haaren zu steigern, ohne hierdurch ihre physiologische Verträglichkeit zu verschlechtern.

Es wurde nunmehr gefunden, daß die Wirkung von kosmetischen Mitteln zur dauerhaften Verformung oder zur Entfernung von Haaren auf der Basis eines keratinerweichenden oder oxidierenden Wirkstoffes durch den Zusatz einer Mischung aus Siliciumdioxid und einem Dimethylpolysiloxan sowohl im sauren als auch im alkalischen pH-Bereich gesteigert werden kann, ohne daß hierdurch die physiologische Verträglichkeit verschlechtert wird.

Die gemeinsame Verwendung von Dimethylpolysiloxanen und Siliciumdioxid in Haarspülmitteln ist aus der deutschen Offenlegungsschrift 32 06 448 bekannt. Ferner ist aus der deutschen Auslegeschrift 1 017 333 die alleinige Verwendung von Dimethylpolysiloxanen, das heißt ohne Zusatz von Siliciumdioxid, in Haarverformungsmitteln bekannt.

Ein kosmetisches Mittel zur dauerhaften Verformung oder zur Entfernung von Haaren, welches eine Mischung aus Siliciumdioxid und einem Dimethylpolysiloxan enthält, wurde jedoch bisher nicht vorgeschlagen. Ebenfalls ist der wirkungssteigernde Effekt des Zusatzes einer derartigen Mischung zu Haarverformungs- oder Enthaarungsmitteln aus der Literatur nicht bekannt. In der Literatur (vergleiche die deutsche Auslegeschrift 1 017 333) wird vielmehr darauf hingewiesen, daß durch den Zusatz von Dimethylpolysiloxanen zu Haarverformungsmitteln eine Überkrausung der Haare vermieden wird, das heißt, die Wirkung des Verformungsmittels abgeschwächt wird.

Gegenstand der vorliegenden Erfindung ist daher ein kosmetisches Mittel zur dauerhaften Verformung oder zur Entfernung von Haaren auf der Basis eines keratinerweichenden oder eines oxidierenden Wirkstoffes, welches dadurch gekennzeichnet ist, daß es 0,01 bis 1,2 Gewichtsprozent, vorzugsweise 0,03 bis 0,5 Gewichtsprozent, einer Mischung aus Siliciumdioxid mit einer Korngröße von 1 bis 100 mμ und einem Dimethylpolysiloxan der Formel (I)

$$CH_3-\overset{\displaystyle CH_3}{\underset{\displaystyle CH_3}{\overset{|}{\underset{|}{Si}}}}-O-\left[\overset{\displaystyle CH_3}{\underset{\displaystyle CH_3}{\overset{|}{\underset{|}{Si}}}}-O-\right]_n-\overset{\displaystyle CH_3}{\underset{\displaystyle CH_3}{\overset{|}{\underset{|}{Si}}}}-CH_3 \qquad (I),$$

mit n gleich einer ganzen Zahl von 200 bis 350, enthält, wobei der Gehalt an Siliciumdioxid, bezogen auf die Gesamtmenge der Siliciumdioxid/Dimethylpolysiloxan-Mischung, 2 bis 16,5 Gewichtsprozent beträgt.

Bevorzugt besitzt das Siliciumdioxid eine Korngröße von etwa 5 bis 40 mμ. Der Gehalt an Siliciumdioxid in dem erfindungsgemäßen Mittel beträgt, bezogen auf die Gesamtmenge der Siliciumdioxid/Dimethylpolysiloxan-Mischung, vorzugsweise etwa 6,5 bis 12 Gewichtsprozent.

Das erfindungsgemäße kosmetische Mittel liegt in Form eines Mittels zur Durchführung der ersten Stufe einer dauerhaften Haarverformung, eines Mittels zur Entfernung der Haare oder eines Mittels zur Durchführung der zweiten Stufe einer dauerhaften Haarverformung vor. Die Zusammensetzung dieser Mittel entspricht den für derartige Mittel jeweils bekannten Zubereitungsarten auf der Basis von keratinerweichenden oder oxidierenden Wirkstoffen. Die Siliciumdioxid/Dimethylpolysiloxan-Mischung wird hierbei vorzugsweise

in Form einer 30-prozentigen wäßrigen Dispersion eingesetzt.

Diese üblichen Zubereitungsarten, die im Rahmen der Erfindung verwendbar sind, sollen nachstehend erläutert werden.

Bei dem Mittel zur Durchführung der ersten Stufe einer dauerhaften Haarverformung, und zwar sowohl zur Wellung als auch zur Entkräuselung, handelt es sich insbesondere um eine wäßrige, alkalisch (pH = 7 bis 10) eingestellte Zubereitung auf der Basis eines keratinerweichenden Wirkstoffes, die als keratinerweichenden Wirkstoff eine keratinreduzierend wirkende Verbindung, wie zum Beispiel Cystein, N-Acetyl-L-cystein, Thioglycerin, Cysteamin oder Salze von Mercaptocarbonsäuren, wie beispielsweise Ammonium- oder Guanidinsalze der Thioglykolsäure oder Thiomilchsäure, in einer Konzentration von etwa 2 bis 12 Gewichtsprozent enthält. Die erforderliche Alkalität wird hierbei durch die Zugabe von Ammoniak, organischen Aminen, Ammonium-und Alkalicarbonat oder Ammonium-und Alkalihydrogencarbonat eingestellt. Es kommt aber auch ein neutral oder sauer eingestelltes Haarverformungsmittel (pH = 6,5 bis 7,0) in Betracht, das in wäßrigem Medium einen wirksamen Gehalt an Sulfiten oder Mercaptocarbonsäureestern aufweist.

Im ersten Falle werden vorzugsweise Natrium-oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als $SO_2$) verwendet. Im letzteren Falle kommen insbesondere Monothioglykolsäureglycolester oder -glycerinester in einer Konzentration von 5 bis 30 Gewichtsprozent (entsprechend einem Gehalt an freier Thioglykolsäure von 2 bis 16 Gewichtsprozent) zur Anwendung. Ferner ist ein wäßriges, alkalisches Entkräuselungsmittel auf der Basis einer starken Base, wie zum Beispiel Lithium-, Natrium-oder Kaliumhydroxid, die in einer Menge von etwa 2 bis 12 Gewichtsprozent enthalten ist, verwendbar.

In dem Mittel zur Durchführung der ersten Stufe einer dauerhaften Haarverformung kann auch ein Gemisch der vorstehend genannten keratinerweichenden Wirkstoffe enthalten sein.

Handelt es sich bei dem erfindungsgemäßen Mittel um ein Mittel Zur Entfernung der Haare, so liegt es als wäßrige, stark alkalische (pH = 11,0 bis 12,5) Zubereitung auf der Basis eines keratinerweichenden Wirkstoffes vor. Als keratinerweichenden Wirkstoff enthält ein derartiges Mittel vorzugsweise eine keratinerweichende Verbindung aus der Gruppe der Sulfide, wie beispielsweise Strontiumsulfid, oder ein Salz einer Mercaptocarbonsäure, wie zum Beispiel Natrium-und Calciumthioglykolat oder Natrium-und Calciumthiolactat, in einer Menge von 4 bis 10 Gewichtsprozent. Zur Einstellung des erforderlichen pH-Wertes verwendet man hierbei vorzugsweise Calcium-oder Strontiumhydroxid sowie Calcium-oder Magnesiumcarbonat in einer Menge von etwa 1 bis 20 Gewichtsprozent.

Bei dem Mittel zur Durchführung der zweiten Stufe der Haarverformung handelt es sich vo eine wäßrige, sauer eingestellte Zubereitung auf der Basis eines oxidierenden Wirkstoffes. Der oxidierende Wirkstoff, vorzugsweise Hydrogenperoxid oder Alkalibromate, wie zum Beispiel Kalium-und Natriumbromat, ist in diesem Mittel in einer Menge von 0,5 bis 10 Gewichtsprozent enthalten. Ebenfalls kann dieses Mittel weitere für ein derartiges Mittel übliche Zusätze, wie beispielsweise Säuren oder Peroxidstabilisatoren, enthalten.

Sowohl das auf einem keratinerweichenden Wirkstoff basierende Mittel zur dauerhaften Haarverformung oder zur Entfernung von Haaren als auch das auf einem oxidierenden Wirkstoff basierende Mittel zur dauerhaften Haarverformung kann in Form einer wäßrigen Lösung oder Emulsion sowie in verdickter Form auf wäßriger Basis, insbesondere als Creme, Gel oder Paste, vorliegen.

Selbstverständlich kann sowohl das auf einem keratinerweichenden Wirkstoff basierende Mittel zur dauerhaften Haarverformung oder zur Entfernung von Haaren als auch das auf einem oxidierenden Wirkstoff basierende Mittel zur dauerhaften Haarverformung alle für ein derartiges Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oöer Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von etwa 0,2 bis 10 Gewichtsprozent, während die Verdickungsmittel in einer Menge von etwa 1,0 bis 25 Gewichtsprozent in diesem Mittel enthalten sein können.

Weiterhin können diesem Mittel zusätzlich bekannte wirkungsverstärkende Stoffe, wie zum Beispiel

Dipropylenglykolmonomethylether oder 2-Pyrrolidon, in einer Menge von etwa 2 bis 30 Gewichtsprozent zugesetzt werden.

Die als wirkungsverstärkender Zusatz in dem erfindungsgemäßen Mittel enthaltene Siliciumdioxid/Dimethylpolysiloxan-Mischung ist physiologisch verträglich und gegen Reduktion, Oxidation und Verseifung beständig.

Durch den Zusatz der Siliciumdioxid/Dimethylpolysiloxan-Mischung zu dem beschriebenen Haarverformungsmittel auf der Basis eines keratinerweichenden Wirkstoffes wird die Wirkung dieses Mittels wesentlich gesteigert. Die hierdurch mögliche Verringerung der erforderlichen Menge an keratinerweichendem Wirkstoff und/oder des pH-Wertes (auf 6,5 bis 8,0) gestattet eine schnelle und zugleich schonende Haarverformungsbehandlung.

Der wirkungssteigernde Effekt der Siliciumdioxid/Dimethylpolysiloxan-Mischung ist besonders stark bei einem Verformungsmittel, das als keratinerweichenden Wirkstoff Cystein, ein Sulfit, ein Salz der Thioglykolsäure oder einen Ester der Thioglykolsäure enthält. Der pH-Wert dieses Mittels liegt vorzugsweise bei etwa 6,5 bis 9,5.

Wie Vergleichsversuche gezeigt haben, kann in einem erfindungsgemäßen Verformungsmittel, welches Ammoniumthioglykolat oder Ammoniumsulfit als keratinerweichendem Wirkstoff enthält, der Wirkstoffgehalt bei Zusatz von 0,3 Prozent der Siliciumdioxid/Dimethylpolysiloxan-Mischung um über 10 Prozent gesenkt werden, ohne daß die Verformungswirksamkeit dieses Mittels hierdurch verringert wird.

Ebenfalls ermöglicht der Zusatz der Siliciumdioxid/Dimethylpolysiloxan-Mischung bei einem Mittel zur dauerhaften Haarverformung auf der Basis eines oxidierenden Wirkstoffes, insbesondere bei einem bromathaltigen Mittel, eine schnellere und zuverlässigere oxidative Fixierung. Der pH-Wert dieses Haarverformungsmittels auf Bromatbasis beträgt vorzugsweise etwa 6,0 bis 7,5.

Bei Verwendung des erfindungsgemäßen Haarverformungsmittels auf der Basis eines keratinerweichenden oder oxidierenden Wirkstoffes werden außerdem Griff, Kämmbarkeit, Glanz, Krausenstärke und Elastizität der Haare deutlich verbessert.

Weiterhin verkürzt sich bei Enthaarungsmitteln auf der Basis eines keratinerweichenden Wirkstoffes durch den Zusatz der Siliciumdioxid/Dimethylpolsiloxan-Mischung die für eine Entfernung der Haare erforderliche Einwirkungszeit um etwa 10 Prozent. Hierdurch kann die Gefahr einer Hautirritation oder Hautschädigung vermindert werden, da die Haut nicht mehr so lange mit dem agressiven Enthaarungsmittel in Kontakt kommt.

Die praktische Durchführung der dauerhaften Haarverformung erfolgt im allgemeinen in der Weise, daß man das Haar wäscht, mit einem Handtuch frottiert, gegebenenfalls mit einem Teil des vorstehend beschriebenen Haarverformungsmittels auf der Basis eines keratinreduzierenden Wirkstoffes vorfeuchtet, in einzelne Strähnen aufteilt und auf Wickler wickelt. Der Durchmesser der Wickler beträgt hierbei, je nachdem ob eine Dauerwellung oder eine Haarentkräuselung gewünscht wird, entweder etwa 5 bis 13 Millimeter oder etwa 15 bis 35 Millimeter. Auf das aufgewickelte Haar wird anschließend eine für die Haarverformung ausreichende Menge des Verformungsmittels aufgetragen. Die für eine Haarverformung erforderliche Gesamtmenge des Verformungsmittels beträgt hierbei im allgemeinen 80 bis 120 Gramm.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 5 bis 30 Minuten beträgt, wird das Verformungsmittel mit Wasser ausgespült und das Haar in einer zweiten Verfahrensstufe oxidativ fixiert. Das Haarverformungsmittel auf der Basis eines oxidierenden Wirkstoffes wird hierbei in einer Menge von etwa 80 bis 100 Gramm verwendet.

Für die Fixierung kann jeder beliebige, bisher in Haarverformungsmitteln verwendete oxidierende Wirkstoff eingesetzt werden. Beispiele für solche oxidierenden Wirkstoffe sind Alkalibromate, wie zum Beispiel Kalium-und Natriumbromat, oder Wasserstoffperoxid. Die Konzentration des oxidierenden Wirkstoffes ist in Abhängigkeit von der Anwendungsdauer und Anwendungstemperatur unterschiedlich. Normalerweise wird er in einer Konzentration von etwa 0,5 bis 10 Gewichtsprozent verwendet.

Nach einer Einwirkungszeit von 5 bis 15 Minuten werden die Wickler entfernt, das Haar mit Wasser ausgespült und in üblicher Weise weiterbehandelt. In der Regel wird das Haar im Anschluß an eine Dauerverformung zur Wasserwelle gelegt. Im Falle einer Haarentkräuselung kann auch so verfahren werden, daß man das oxidierend wirkende Verformungsmittel bei gewickeltem Haar abspült und das Haar anschließend, ohne abzuwickeln, direkt am Wickler trocknet.

Bei dem vorstehend beschriebenen Verfahren zur dauerhaften Haarverformung kann für die zweite, oxidierende Stufe jedes übliche Haarverformungsmittel auf der Basis eines oxidierenden Wirkstoffes verwendet werden. Besonders vorteilhaft ist es jedoch, ein Haarverformungsmittel auf der Basis eines oxidierenden Wirkstoffes gemäß der vorliegenden Erfindung zu verwenden.

Bei der Haarentkräuselung ist es ebenfalls möglich, in dem erfindungsgemäßen Mittel anstelle von keratinreduzierenden Schwefelverbindungen starke Basen, wie beispielsweise Lithium-, Natrium-, Kalium-, Guanidin-oder Tetraalkylammoniumhydroxid als keratinerweichenden Wirkstoff einzusetzen, wobei dann das Haar ohne die Anwendung von Wicklern durch mehrmaliges Kämmen während der Einwirkungszeit geglättet wird. Anschließend wird das Haar mit lauwarmem Wasser gründlich gespült und sodann getrocknet.

Die Entfernung von Haaren erfolgt im allgemeinen indem man ein Enthaarungsmittel, vorzugsweise in Form einer Creme oder Paste, auf das zu behandelnde Haar so aufbringt, daß die Haare von dem Enthaarungsmittel vollständig bedeckt werden. Nach einer Einwirkungszeit von etwa 3 bis 10 Minuten wird das Enthaarungsmittel wieder entfernt und die Haut mit lauwarmem Wasser abgespült.

Die nachfolgenden Beispiele sollen den Gegenstand der Erfindung näher erläutern, ohne diesen hierauf zu beschränken.

## Beispiele

### Kosmetische Mittel auf der Basis eines keratinerweichenden Wirkstoffes

**Beispiel 1:** Dauerwellmittel auf Thioglykolatbasis

| | |
|---|---|
| 0,11 g | Siliciumdioxid-Dimethylpolysiloxan-Mischung (Dimethylpolysiloxan der allgemeinen Formel (I), mit n = 250, in dem 10 Gewichtsprozent Siliciumdioxidpulver mit einer Korngröße von 20 mμ enthalten sind) |
| 15,00 g | Ammoniumthioglykolat (70-prozentige wäßrige Lösung) |
| 5,00 g | Ammoniumcarbonat |
| 2,00 g | Ammoniumhydrogencarbonat |
| 0,30 g | 1,4-Nonylphenol mit 10 Mol Ethylenoxid oxethyliert |
| 0,10 g | Parfümöl |
| 77,49 g | Wasser |
| 100,00 g | |

Der pH-Wert dieses Mittels beträgt 9,0.

Das Haar wird mit einem milden Shampoo gewaschen. Anschließend wird das handtuchtrockene Haar mit etwa der Hälfte der vorstehend aufgeführten Wellflüssigkeit vorgefeuchtet, auf Dauerwellwickler mit einem Durchmesser von 8 Millimetern gewickelt und sodann mit der restlichen Dauerwellflüssigkeit nachgefeuchtet. Anschließend werden die Haare mit einer Plastikfolie abgedeckt. Nach 15 Minuten wird das gewickelte Haar mit Wasser gründlich gespült und sodann mit 80 Gramm einer 3-prozentigen Hydrogenperoxidlösung oxidativ nachbehandelt. Nach Entfernung der Wickler werden die Haare erneut mit Wasser gespült und anschließend getrocknet.

Das so behandelte Haar besitzt eine gleichmäßige, elastische Krause

**Beispiel 2:** Dauerwellmittel auf Sulfitbasis

| | |
|---|---|
| 0,4 g | 30-prozentige wäßrige Silicium-dioxid/Dimethylpolysiloxan-Dispersion (Dimethylpolysiloxan der allgemeinen Formel (I), mit n = 300, in dem 10 Gewichtsprozent Siliciumdioxidpulver mit einer Korngröße von 40 mμ enthalten sind) |
| 20,0 g | Ammoniumsulfit (34-prozentige wäßrige Lösung) |
| 15,6 g | schweflige Säure (mit 5 Prozent $SO_2$-Gehalt) |
| 4,0 g | Dipropylenglykolmonomethylether |
| 0,2 g | Octylphenol, mit 20 Mol Ethylenoxid oxethyliert |
| 0,1 g | Parfümöl |
| 59,7 g | Wasser |
| 100,0 g | |

Der pH-Wert dieses Mittels beträgt 6,7.

Die Haare werden mit etwa der Hälfte der obigen Dauerwellflüssigkeit vorgefeuchtet, auf Wickler mit einem Durchmesser von 10 Millimetern gewickelt und sodann mit der restlichen Dauerwellflüssigkeit nachgefeuchtet. Anschließend deckt man die Haare mit einer Plastikhaube ab und erwärmt die Haare mit einem Infrarotstrahler 10 Minuten lang auf 40 Grad Celsius. Anschließend werden die Haare mit Wasser gespült, sodann mit 100 g einer 3-prozentigen wäßrigen Hydrogenperoxidlösung oxidativ fixiert und wie üblich nachbehandelt.

Das so behandelte Haar besitzt eine gleichmäßige Krause, deren Locken eine große Elastizität und Sprungkraft sowie einen starken Glanz besitzen.

**Beispiel 3:** Dauerwellmittel auf Thioglykolsäureesterbasis

Komponente (A):

| | |
|---|---|
| 0,3 g | 30-prozentige wäßrige Silicium-dioxid/Dimethylpolsiloxan-Dipersion (Dimethylpolysiloxan der Formel (I), mit n = 300, in dem 10 Gewichtsprozent Silicium-dioxidpulver mit einer Korngröße von 40 mμ enthalten sind) |
| 1,5 g | Ammoniak, 25-prozentige wäßrige Lösung |
| 0,5 g | Diammoniumdithiodilactat |
| 0,5 g | Ammoniumhydrogencarbonat |
| 0,2 g | Parfümöl |
| 97,0 g | Wasser |
| 100,0 g | |

Komponente (B):

| | |
|---|---|
| 30,0 g | Thioglykolsäuremonoglycerinester |
| 20,0 g | Glycerin |
| 50,0 g | |

Das gebrauchfertige Dauerwellmittel wird durch Vermischen der Komponenten (A) und (B) hergestellt. Der pH-Wert des gebrauchsfähigen Mittels beträgt 7,0.

Das gewaschene und handtuchtrockene Haar wird auf Wickler mit einem Durchmesser von 7 Millimetern gewickelt und anschließend mit etwa 120 g des obigen Dauerwellmittels gut durchfeuchtet. Nach einer Einwirkungszeit von 15 Minuten bei Raumtemperatur wird das gewickelte Haar mit Wasser gründlich gespült und sodann mit 80 g eines Fixiermittels gemäß Beispiel 7 oxidativ nachbehandelt. Nach Entfernung der Wickler werden die Haare erneut mit Wasser gespült und danach getrocknet.

Als Ergebnis dieser Behandlung wird ein gleichmäßig verformtes, glänzendes Haar mit hoher Sprungkraft und Elastizität erhalten.

**Beispiel 4:** Haarentkräuselungscreme auf Thioglykolatbasis

| | |
|---|---|
| 0,34 g | Siliciumdioxid/Dimethylpolysiloxan-Mischung (Dimethylpolysiloxan der Formel (I), mit n = 350, in dem 12 Gewichtsprozent Siliciumdioxidpulver mit einer Korngröße von 10 mμ enthalten sind) |
| 15,00 g | Ammoniumthioglykolat, 70-prozentige wäßrige Lösung |
| 7,00 g | Cetylalkohol |
| 4,00 g | Ammoniak, 25-prozentige wäßrige Lösung |
| 2,00 g | Natriumlaurylethersulfat |
| 0,50 g | Parfümöl |
| 71,16 g | Wasser |
| 100,00 g | |

Die Haarentkräuselungscreme besitzt einen pH-Wert von 9,5.

Man trägt die obige Haarentkräuselungscreme gleichmäßig auf das krause Haar auf. Sodann wird das Haar während einer Einwirkungszeit von etwa 15 Minuten mehrmals glattgekämmt und anschließend gründlich mit Wasser ausgespült. Sodann wird das Haar mit 100 g einer Natriumbromatlösung gemäß Beispiel 6 oxidativ fixiert, erneut mit Wasser gespült und danach getrocknet.

**Beispiel 5:** Enthaarungsmittel auf Thioglykolatbasis

| | |
|---|---|
| 0,055 g | Siliciumdioxid/Dimethylpolysiloxan-Mischung (Dimethylpolysiloxan der Formel (I), mit n = 300, in dem 9 Gewichtsprozent Siliciumdioxidpulver mit einer Korngröße von 30 mμ enthalten sind) |
| 10,000 g | Calciumhydroxid |
| 6,000 g | Natriumthioglykolat, 50-prozentige wäßrige Lösung |
| 5,000 g | Stearylalkohol |
| 1,000 g | Natriumlaurylsulfat |
| 0,400 g | Parfümöl |
| 77,545 g | Wasser |
| 100,000 g | |

Der pH-Wert der Enthaarungscreme beträgt 12,0.

Der pH-Wert der Enthaarungscreme beträgt 12,0

Die obige Enthaarungscreme wird mit einem Spatel so aufgetragen, daß alle zu entfernenden Haare gleichmäßig von ihr bedeckt werden. Nach einer Einwirkungszeit von etwa 6 Minuten wird die Enthaarungscreme mit einem Spatel entfernt. Anschließend wird die Haut zur Entfernung von Enthaarungscremeresten mit 35 Grad Celsius warmem Wasser gründlich abgewaschen.

**Kosmetische Mittel auf der Basis eines oxidierenden Wirkstoffes**

**Beispiel 6:** Bromathaltiges Haarverformungsmittel

| | |
|---|---|
| 1,1 g | Siliciumdioxid/Dimethylpolysiloxan-Mischung (Dimethylpolysiloxan der Formel (I), mit n = 200, in dem 10 Gewichtsprozent Siliciumdioxidpulver mit einer Korngröße von 20 mμ enthalten sind) |
| 5,0 g | Natriumbromat |
| 1,0 g | Isooctylphenol, mit 10 Mol Ethylenoxid oxethyliert |
| 0,2 g | Parfümöl |
| 92,7 g | Wasser |
| 100,0 g | |

Der pH-Wert der Lösung beträgt 6,5.

Nach dem Abspülen eines Haarverformungsmittels auf der Basis eines keratinreduzierenden Wirkstoffes werden etwa 80 g des obigen oxidierend wirkenden Haarverformungsmittels auf das Haar aufgebracht. Nach einer Einwirkungszeit von etwa 5 Minuten wird abgewickelt und die restliche Flüssigkeitsmenge (20 Gramm) gleichmäßig auf das Haar verteilt. Nach etwa 3 Minuten wird das Haar erneut mit Wasser gespült und sodann getrocknet.

**Beispiel 7:** Hydrogenperoxidhaltiges Haarverformungsmittel

| | |
|---|---|
| 0,22 g | Siliciumdioxid/Dimethylpolysiloxan-Mischung (Dimethylpolysiloxan der Formel (I), mit n = 300, in dem 9 Gewichtsprozent Siliciumdioxidpulver mit einer Korngröße von 40 mμ enthalten sind) |
| 5,00 g | Hydrogenperoxid, 50-prozentige wäßrige Lösung |
| 1,50 g | Kokosfettsäuredimethylammoniumbetain |
| 0,20 g | Parfümöl |
| 93,08 g | Wasser |
| 100,00 g | |

Die Anwendung erfolgt gemäß Beispiel 6.

Alle in der vorliegenden Anmeldung enthaltenen Prozentangaben stellen Gewichtsprozente dar.

## Ansprüche

1. Kosmetisches Mittel zur dauerhaften Verformung oder zur Entfernung von Haaren auf der Basis eines keratinerweichenden oder eines oxidierenden Wirkstoffes, dadurch gekennzeichnet, daß es 0,01 bis 1,2 Gewichtsprozent einer Mischung aus Siliciumdioxid mit einer Korngröße von 1 bis 100 mµ und einem Dimethylpolysiloxan der Formel (I)

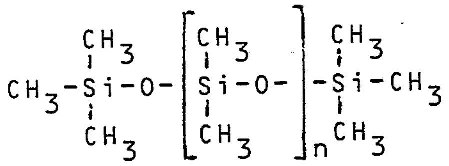

(I),

mit n gleich einer ganzen Zahl von 200 bis 350, enthält, wobei der Gehalt an Siliciumdioxid, bezogen auf die Gesamtmenge der Siliciumdioxid/Dimethylpolysiloxan-Mischung, 2 bis 16,5 Gewichtsprozent beträgt.

2. Mittel nach Anspruch 1, dadurch gekennzeichnet, daß es die Siliciumdioxid/Dimethylpolysiloxan-Mischung in einer Menge von 0,03 bis 0,5 Gewichtsprozent enthält.

3. Mittel nach Anspruch 1 oder 2, dadurch gekennzeichnet, daß das Siliciumdioxid eine Korngröße von 5 bis 40 mµ besitzt.

4. Mittel nach einem der Ansprüche 1 bis 3, dadurch gekennzeichnet, daß der Gehalt an Siliciumdioxid, bezogen auf die Gesamtmenge der Siliciumdioxid/Dimethylpolysiloxan-Mischung, 6,5 bis 12 Gewichtsprozent beträgt.

5. Mittel nach einem der Ansprüche 1 bis 4, dadurch gekennzeichnet, daß der keratinerweichende Wirkstoff ein Salz oder Ester einer Mercaptocarbonsäure, Cystein, N-Acetyl-L-cystein, Cysteamin, Thioglycerin, ein Sulfit, ein Sulfid oder eine starke Base ist.

6. Mittel zur dauerhaften Verformung von Haaren nach einem der Ansprüche 1 bis 5, dadurch gekennzeichnet, daß es den keratinerweichenden Wirkstoff in einer Menge von 2 bis 12 Gewichtsprozent enthält.

7. Mittel zur dauerhaften Verformung von Haaren nach einem der Ansprüche 1 bis 4, dadurch gekennzeichnet, daß der oxidierende Wirkstoff ausgewählt ist aus Hydrogenperoxid und Alkalibromat.

8. Mittel zur dauerhaften Verformung von Haaren nach Anspruch 7, dadurch gekennzeichnet, daß es den oxidierenden Wirkstoff in einer Menge von 0,5 bis 10 Gewichtsprozent enthält.

9. Mittel zur Entfernung von Haaren nach einem der Ansprüche 1 bis 5, dadurch gekennzeichnet, daß es den keratinerweichenden Wirkstoff in einer Menge von 4 bis 10 Gewichtsprozent enthält.

10. Mittel zur Entfernung von Haaren nach Anspruch 9, dadurch gekennzeichnet, daß es einen pH-Wert von 11,0 bis 12,5 besitzt

Europäisches Patentamt

# EUROPÄISCHER RECHERCHENBERICHT

Nummer der Anmeldung

EP 88 10 2552

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE-A-3 206 448 (KAO SOAP)<br>* Ansprüche; Seite 3, Zeile 1 - Seite 5, Zeile 3; Beispiele 1,2 *<br>--- | 1-10 | A 61 K 7/09<br>A 61 K 7/155 |
| P,A | EP-A-0 240 350 (PROCTER & GAMBLE CO.)<br>* Ansprüche; Beispiele 1,5,10 *<br>--- | 1-10 | |
| P,A | EP-A-0 240 349 (PROCTER & GAMBLE)<br>* Ansprüche; Seite 1, Zeilen 3-35 *<br>----- | 1-10 | |
| | | | **RECHERCHIERTE SACHGEBIETE (Int. Cl.4)** |
| | | | A 61 K 7/00 |

Der vorliegende Recherchenbericht wurde für alle Patentansprüche erstellt

| Recherchenort | Abschlußdatum der Recherche | Prüfer |
|---|---|---|
| DEN HAAG | 22-07-1988 | WILLEKENS G.E.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)